Europäisches Patentamt

European Patent Office

Office européen des brevets

(11) Veröffentlichungsnummer: **0 216 194 B1**

## EUROPÄISCHE PATENTSCHRIFT

(45) Veröffentlichungstag der Patentschrift: **08.07.92**

(21) Anmeldenummer: **86111995.6**

(22) Anmeldetag: **29.08.86**

Die Akte enthält technische Angaben, die nach
dem Eingang der Anmeldung eingereicht wurden
und die nicht in dieser Patentschrift enthalten sind.

(51) Int. Cl.⁵: **G01N 33/53**, G01N 33/543,
C12Q 1/34

(54) **Verfahren und Reagenz zur Beseitigung der Störung der Analyt-Wiederfindung in Plasmaproben.**

(30) Priorität: **29.08.85 DE 3530942**

(43) Veröffentlichungstag der Anmeldung:
**01.04.87 Patentblatt 87/14**

(45) Bekanntmachung des Hinweises auf die
Patenterteilung:
**08.07.92 Patentblatt 92/28**

(84) Benannte Vertragsstaaten:
**AT BE CH DE FR GB IT LI LU NL SE**

(56) Entgegenhaltungen:

**CHEMICAL ABSTRACTS, Band 103, Nr. 5, 05
August 1985, Columbus, OH (US); R.ALLNER,
Seite 239, Nr. 34357g**

**H.U. BERGMEYER, "Methods of Enzymatic
Analysis", 3. Auflage, Band V, 1984, Verlag
Chemie, Weinheim (DE); J.H.VERHEIJEN et
al., Seite 425**

**PROGRESS IN FIBRINOLYSIS, Band VII, Kapitel 9 und 10, 1985**

(73) Patentinhaber: **BOEHRINGER MANNHEIM
GMBH
Sandhofer Strasse 116
W-6800 Mannheim 31(DE)**

(72) Erfinder: **Deeg, Rolf, Dr.
Hirtenstr. 7
W-8139 Bernried(DE)**
Erfinder: **Wehner, Rainer, Dr.
Alpspitzstr. 6
W-8132 Tutzing(DE)**
Erfinder: **Mattersberger, Johann, Dr.
Oefelestr. 5
W-8000 München 90(DE)**
Erfinder: **Becker, Udo, Dr.
Birkenweg 2
W-3550 Marburg 1(DE)**

(74) Vertreter: **Weickmann, Heinrich, Dipl.-Ing. et
al
Patentanwälte H.Weickmann, Dr. K.Fincke
F.A. Weickmann, B. Huber Dr. H. Liska, Dr. J.
Prechtel Kopernikusstrasse 9 Postfach 86 08
20
W-8000 München 86(DE)**

CLINICAL HEMATOLOGY, 1974; WINTROBE et al., Seiten 434-435

J. CLIN. CHEM., CLIN. BIOCHEM., Band 23, 1985; Seiten 231-240

EP 0 216 194 B1

**Beschreibung**

Die Erfindung betrifft ein Verfahren zur Bestimmung eines Partners einer Immunreaktion in einer Plasmaprobe nach dem Immunoassay-Prinzip bei einer Temperatur zwischen 15 und 40 °C, wobei einer der Reaktionspartner in fester Phase vorliegt.

Verfahren zur Immunbestimmung sind weit verbreitet. Dabei gibt es sowohl Verfahren in homogener als auch in heterogener Phase. Bei der Ausführungsform mit der heterogenen Phase wird einer der Reaktionspartner an einen Träger gebunden. Zur Durchführung der Immunbestimmung in heterogener Phase sind nun verschiedene Verfahrensvarianten bekannt, z. B. das Sandwich-Verfahren, das indirekte Verfahren und das Kompetitionsverfahren. Beim Sandwich-Verfahren wird ein Antikörper an den Träger gebunden, die Testlösung wird zugegeben, wobei das in der Testlösung enthaltene spezifische Antigen an den Antikörper gebunden wird. Dann wird ein markierter spezifischer Antikörper für den Antigen-Antikörper-Komplex, oder für Teile des Komplexes, zugegeben, der dann an den Komplex bindet. Über den markierten Antikörper kann man dann die Menge an Antigen berechnen. Beim indirekten Verfahren wird ein Antigen an dem Trägermaterial gebunden. Man gibt Testlösung dazu, wobei der in der Testlösung enthaltene, für das gebundene Antigen spezifische Antikörper mit dem Antigen reagiert. Bei Zugabe eines markierten Antiglobulins bindet das Antiglobulin an den Antigen-Antikörper-Komplex und die Menge des unbekannten Antikörpers im Testserum kann wiederum über das markierte Antiglobulin bestimmt werden. Bei dem Kompetitionsverfahren wird einer der gebundenen Partner der Immunreaktion an das Trägermaterial gebunden. Man gibt dann eine Lösung dazu, die sowohl den in unbekannter Menge vorliegenden anderen Partner der Immunreaktion enthält, als auch eine bekannte Menge an markiertem anderem Partner der Immunreaktion. Beide Partner, der markierte und der unmarkierte, konkurrieren um die Bindungsstellen des am Träger gebundenen Partners der Immunreaktion. Zu einer zweiten Probe gibt man eine Standardlösung dazu, die nur markierten Partner enthält. Man kann nun durch Bestimmung der markierten Partner in Standard und Probe aus der Differenz die Menge an unbekanntem Reaktionspartner berechnen.

Weitere Möglichkeiten zur Bestimmung ergeben sich bei einem mindestens bivalenten Antigen mit Hilfe von drei Rezeptoren, die Antikörper oder Antikörperfragmente sein können. Bei der Durchführung eines Immunoassays mit drei Rezeptoren in heterogener Phase ist einer der drei Rezeptoren immer unlöslich, während die anderen zwei löslich sind, wobei einer der beiden löslichen Rezeptoren markiert ist, während der andere nicht markiert ist. Der unlösliche Rezeptor ist dann gegen den nicht markierten löslichen Rezeptor gerichtet. Zur Durchführung der Bestimmung sind verschiedene Verfahrensvarianten möglich, sowohl einstufige, als auch mehrstufige, in denen die Zugabe der einzelnen Rezeptoren variiert werden kann.

So besteht eine weitere Verfahrensvariante darin, das zu bestimmende Antigen in der Probe im ersten Schritt gleichzeitig mit einem löslichen, nicht markierten Rezeptor und einem löslichen markierten Rezeptor in Lösung umzusetzen und dann den entstehenden löslichen Sandwich-Komplex in einem zweiten Schritt durch Binden an einen unlöslichen Rezeptor unlöslich zu machen.

Zur Markierung der Reaktionspartner sind verschiedene Möglichkeiten bekannt. So kann einer der Reaktionspartner radioaktiv markiert werden, wobei dann die gemessene Radioaktivität eine Berechnung des zu bestimmenden Reaktionspartners erlaubt. Weiterhin kann mit einem Enzym markiert werden. Es wird dann nach Durchführung der üblichen Schritte der Immunbestimmung ein Substrat für das Enzym, das als Markierung verwendet wird, zugegeben und dann über dessen Reaktion die Konzentration des zu bestimmenden Reaktionspartners berechnet. Eine weitere Möglichkeit ist die Markierung mit einer fluoreszierenden Substanz, wobei die Fluoreszenz dann direkt bestimmt werden kann oder mit einem Farbstoff, wobei die Auswertung spektro- oder photometrisch erfolgt.

Immunoassays weisen wachsende Bedeutung auf, da mit Hilfe dieses Verfahrens einerseits Substanzen sehr spezifisch nachgewiesen werden können und sie andererseits eine sehr große Empfindlichkeit haben und mit Hilfe dieser Methoden Substanzen bis in den Picogrammbereich erfaßt werden können. Immunbestimmungen in heterogener Phase werden jedoch in unterschiedlichem Ausmaß durch nicht analytspezifische Interferenzen, sogenannte unspezifische Störungen, verfälscht, die schon als "Matrixeffekt", "Background" und "unspezifische Bindung" bezeichnet wurden. Besonders störend macht sich bemerkbar, daß die Wiederfindung der zu bestimmenden Substanz in Plasmaproben bei Sandwichverfahren deutlich geringer ist als bei Serumproben und bei kompetitiven Verfahren deutlich höher ist als bei Serumproben. Es wurde nun gefunden, daß diese Verfälschung der Ergebnisse durch Fibrinogen, das im Plasma vorhanden ist, im Serum jedoch nicht, verursacht wird. Diese Störung ist unabhängig von der Art der Stabilisierung des Plasmas, d.h. zugegebenes EDTA, Citrat, Heparin etc. haben keinen Einfluß auf die Wiederfindung. Sie tritt jedoch nur bei Immunbestimmungen in heterogener Phase auf. Es wurde nun festgestellt, daß bei Durchfuhrung eines kompetitiven Verfahrens Fibrinogen den zu bestimmenden Analyten

3

EP 0 216 194 B1

vortäuscht. Dies geschieht nicht durch Kreuzreaktion, sondern dadurch, daß sich Fibrinogen als oberflächenaktive Substanz auf den Oberflächen des Trägermaterials, an denen der in fester Phase vorliegende Partner der Immunreaktion gebunden ist, festsetzt und somit zum Teil den gebundenen Partner überdeckt. Andererseits führt das Fibrinogen bei Sandwich-Verfahren zu erniedrigten Werten. Um dieses Problem zu lösen, wurde in J.Clin. Chem.Clin.Biochem. Vol. 23 (1985), 231-240 von Allner schon vorgeschlagen, Plasmaproben vor Durchführung der Immunbestimmung 10 Minuten lang im Wasserbad auf 56°C zu erhitzen und anschließend zu zentrifugieren. Dieses Verfahren ist einerseits aufwendiger, andererseits ist es nur dann anwendbar, wenn Substanzen bestimmt werden, die durch diese Behandlung nicht denaturiert werden. Es wird nun weiterhin vorgeschlagen, durch Harnstoffzusatz den störenden Effekt des Fibrinogens zu beeinflussen. Dabei wurde jedoch gefunden, daß diese Beeinflussung nur unter ganz bestimmten Bedingungen möglich ist und daß diese Bedingungen für jede individuelle Plasmaprobe neu bestimmt werden müssen. Auch dieses Verfahren ist also sehr aufwendig.

Es war daher Ziel der Erfindung, ein Verfahren zu finden, mit Hilfe dessen es möglich ist, einen Partner einer Immunreaktion nach dem Immunoassay-Prinzip zu bestimmen, bei dem einer der Reaktionspartner in fester Phase vorliegt, das auch auf Plasmaproben anwendbar

ist, ohne daß dazu aufwendige Verfahrensschritte notwendig sind. Weiterhin war es ein Ziel der Erfindung, Reagenzien zur Verfügung zu stellen, die für Immunbestimmungen in Plasmaproben eingesetzt werden können.

Diese Ziele werden erreicht durch ein Verfahren zur Bestimmung eines Partners einer Immunreaktion nach dem Immunoassay-Prinzip in Plasmaproben bei einer Temperatur zwischen 15 und 40°C, wobei einer der Reaktionspartner in fester Phase vorliegt, das dadurch gekennzeichnet ist, daß man dem Inkubationsmedium der Immunreaktion ein fibrinolytisch wirksames Mittel zusetzt.

Überraschenderweise wurde festgestellt, daß ein Zusatz eines fibrinolytisch wirksamen Mittels zu dem Inkubationsmedium bei einer Immunbestimmung die Wiederfindung verbessert, so daß die Werte, die aus Plasmaproben erhalten werden mit denen, die aus Serumproben erhalten werden, im wesentlichen übereinstimmen. Dies war überraschend, da diese Verbesserung bei der üblichen Durchführung des Verfahrens, d. h. bei den üblichen Inkubationszeiten und Inkubationstemperaturen auftritt. Aus der Literatur war es bisher bekannt, daß der Abbau des Fibrinogens über fibrinolytisch wirksame Mittel lange Inkubationszeiten und höhere Temperaturen benötigt. Mit dem erfindungsgemäßen Verfahren ist es möglich, ohne Durchführung weiterer Verfahrensschritte durch einfaches Zumischen, die Immunbestimmung in Plasmaproben zu verbessern.

Das Verfahren kann bei allen Plasmaarten angewendet werden. Es eignet sich sowohl für Plasma, das zur Stabilisierung mit EDTA versetzt wurde, als auch für Plasma, das mit Heparin, Citrat, Oxalat, Fluorid oder ähnlichen Substanzen stabilisiert wurde.

Eine Beeinflussung der Immunbestimmung durch die fibrinolytisch wirksamen Mittel tritt nicht auf, so daß durch den erfindungsgemäßen Zusatz keine Verfälschung der Werte erfolgt.

Das erfindungsgemäße Verfahren läßt sich sowohl für einstufige als auch für zweistufige Bestimmungsverfahren anwenden. Dabei ist es bevorzugt, bei zweistufigen Verfahren das fibrinolytische Mittel bereits bei der ersten Inkubation zuzusetzen, da schon bei der ersten Inkubation die Plasmaprobe eingesetzt wird und damit das die Störung verursachende Fibrin bzw. Fibrinogen zugeführt wird.

Bevorzugt wird als fibrinolytisch wirksames Mittel ein Plasminogenaktivator zugesetzt. Durch Einwirkung des Plasminogenaktivators wird das im Plasma vorhandene Plasminogen in Plasmin umgewandelt, das dann das Fibrinogen proteolytisch abbaut.

Besonders bevorzugt wird als Plasminogenaktivator Streptokinase oder Urokinase verwendet. Die Streptokinase wird zweckmäßig in Mengen von 10 bis 300 U/ml, besonders bevorzugt von 40 bis 150 U/ml eingesetzt. Bei geringeren Mengen tritt die Wirkung nicht mehr in vollem Maße auf. Höhere Mengen als 300 U/ml können die Wiederfindung nicht weiter verbessern und sind daher unwirtschaftlich.

Die Urokinase wird zweckmäßig in Mengen von 10 bis 120 U/ml, besonders bevorzugt von 20 bis 80 U/ml eingesetzt. Auch hier ist bei geringeren Werten die Wirkung unter Umständen nicht mehr ganz ausreichend, während über dem oberen Grenzwert liegende Konzentrationen unnötig sind.

In einer weiteren bevorzugten Ausführungsform des erfindungsgemäßen Verfahrens wird dem Inkubationsmedium der Immunreaktion als fibrinolytisch wirksames Mittel Extrinsic-Plasminogen-Aktivator (EPA) zugesetzt. EPA wird vorzugsweise in Mengen von 0,2 bis 10 $\mu$g/ml, besonders bevorzugt von 0,5 bis 3 $\mu$g/ml, eingesetzt.

Das erfindungsgemäße Verfahren ist für alle Arten von Immunoassays anwendbar, bei denen einer der Reaktionspartner in fester Phase vorliegt. So ist es geeignet für kompetitive und für Sandwich-Verfahren und für alle Arten von Markierungen.

Zur Durchführung des erfindungsgemäßen Verfahrens ist ein Reagenz geeignet, das neben den an sich

4

üblichen Inhaltsstoffen noch ein fibrinolytisches Mittel enthält. Zweckmäßig enthält das Reagens noch Puffersubstanzen, beispielsweise Phosphatpuffer, Citratpuffer, Boratpuffer und dergleichen oder/und Rinderserumalbumin und/oder Konservierungsmittel. Wird als Markierung ein Enzym verwendet, so enthält das Reagens noch ein System zum Nachweis der Enzymaktivität.

Bevorzugt enthält das erfindungsgemäße Reagenz als fibrinolytisch wirksames Mittel einen Plasminogenaktivator. Als Plasminogenaktivatoren sind bevorzugt Streptokinase, Urokinase und EPA. Das Reagenz enthält bevorzugt 10 bis 300 U/ml, besonders bevorzugt 40 bis 150 U/ml Streptokinase. Weiterhin ist es bevorzugt, 10 bis 120 U/ml, besonders bevorzugt 20 bis 80 U/ml Urokinase dem Reagenz zuzusetzen. Die bevorzugte Menge an EPA beträgt 0,2 bis 10 $\mu$g/ml, besonders bevorzugt werden 0,6 bis 3 $\mu$g/ml zugesetzt.

Die Erfindung soll noch an Beispielen erläutert werden.

**Beispiel 1**

Lösung 1

| Inkubationspuffer | |
| --- | --- |
| Phosphatpuffer | 15 mmol/l, pH 6,9 |
| Rinderserumalbumin (RSA) | 0,2 Gew.-% |
| Merthiolat | 0,01 Gew.-% |

Lösung 2

| Anti-TSH-POD Konjugat gelöst in Lösung 1) | ca. 40 U/l |
| --- | --- |

Lösung 3

| Substrat/Puffer-Lösung | |
| --- | --- |
| Phosphat-Citrat-Puffer | 95 mmol/l, PH 4,4 |
| Natriumperborat | 3,1 mmol/l |
| ABTS$^R$ | 1,6 mmol/l |

2,2'-Azino-di-]3-ethyl-benzthiazolin-sulfonsäure (6)[-diammoniumsalz.

Die verwendeten Lösungen, beschichtete Röhrchen und Standards entstammen dem Enzymun-Test ® TSH (Boehringer Mannheim GmbH, Best.-Nr. 73 60 83). Die Durchführung der Bestimmung erfolgt analog der Vorschrift des Herstellers.

In mit Anti-TSH-Antikörpern beschichteten Röhrchen werden je 0,2 ml TSH-Standard (0 bis 50 $\mu$U/ml) bzw. Probenmaterial (Serum, Plasma) und 1 ml Lösung 1 gegeben und 60 Minuten bei 20 bis 25°C inkubiert. Lösung 1 enthält dabei unterschiedliche Konzentrationen an

Urokinase (0 bis 70 U/ml) bzw. Streptokinase (0 bis 50 U/ml). Nach Aussaugen und Spülen wird 1 ml Lösung 2 zugegeben. Nach 60 Minuten Inkubation bei 20 bis 25°C werden die Röhrchen ausgesaugt und gespült. Anschließend wird 1 ml Lösung 3 zugegeben und wieder 60 Minuten bei 20 bis 25°C inkubiert. Danach wird gegen Lösung 3 als Leerwert eine photometrische Bestimmung bei = 405 nm durchgeführt.

Die in der nachfolgenden Tabelle I aufgeführten Werte sind jeweils bezogen auf den relativen Wert 100 %, der für Serum ohne Zusatz von Urokinase bzw. Streptokinase zum Inkubationspuffer erhalten wurde.

Enzymun-Test ® TSH

Zusatz von Urokinase

| Urokinase-Konz. (U/ml) | Humanserum ( = Referenz) | Heparin-Plasma | EDTA-Plasma |
|---|---|---|---|
| 0 | 100 % | 58 % | 62 % |
| 10 | 96 % | 72 % | 70 % |
| 20 | 96 % | 88 % | 96 % |
| 30 | 97 % | 91 % | 93 % |
| 40 | 94 % | 92 % | 95 % |
| 50 | 99 % | 97 % | 103 % |
| 60 | 100 % | 104 % | 123 % |
| 70 | 86 % | 95 % | 107 % |

Enzymun-Test ® TSH

Zusatz von Streptokinase

| Strepto kinase-Konz. (U/ml) | Humanserum ( = Referenz) | Heparin-plasma | EDTA-Plasma |
|---|---|---|---|
| 0 | 100 % | 49 % | 52 % |
| 0,1 | 98 % | 49 % | 56 % |
| 10 | 103 % | 72 % | 68 % |
| 25 | 94 % | 78 % | 70 % |
| 40 | 106 % | 93 % | 85 % |
| 50 | 106 % | 95 % | 89 % |
| 60 | 100 % | 99 % | 87 % |
| 70 | 106 % | 98 % | 90 % |
| 80 | 100 % | 99 % | 93 % |
| 100 | 102 % | 102 % | 103 % |
| 150 | 100 % | 99 % | 96 % |

**Patentansprüche**

1. Verfahren zur Bestimmung eines Partners einer Immunreaktion in einer Plasmaprobe nach dem Immunoassay-Prinzip bei einer Temperatur zwischen 15 und 40°C, wobei einer der Reaktionspartner in fester Phase vorliegt,
   **dadurch gekennzeichnet,**
   daß man dem Inkubationsmedium der Immunreaktion einen Plasminogenaktivator zusetzt.

2. Verfahren nach Anspruch 1,
   **dadurch gekennzeichnet,**
   daß man als Plasminogenaktivator Streptokinase oder Urokinase verwendet.

3. Verfahren nach Anspruch 2,
   **dadurch gekennzeichnet,**
   daß man 10 bis 300 U/ml, vorzugsweise 40 bis 150 U/ml Streptokinase zusetzt.

4. Verfahren nach Anspruch 2,
   **dadurch gekennzeichnet,**
   daß man 10 bis 120 U/ml, vorzugsweise 20 bis 80 U/ml Urokinase zusetzt.

5. Verfahren nach Anspruch 1,
   **dadurch gekennzeichnet,**
   daß man als Plasminogenaktivator Extrinsic-Plasminogen-Aktivator (EPA) zusetzt.

**6.** Reagenz zur Durchführung des Verfahrens nach einem der Ansprüche 1 bis 5, enthaltend mobilisierte und immobilisierte Partner einer Immunreaktion,
**dadurch gekennzeichnet,**
daß es einen Plasminogenaktivator enthält.

**7.** Reagenz nach Anspruch 6,
**dadurch gekennzeichnet,**
daß es als Plasminogenaktivator Streptokinase, Urokinase oder Extrinsic-Plasminogen-Aktivator (EPA) enthält.

**8.** Reagenz nach Anspruch 7,
**dadurch gekennzeichnet,**
daß es 10 bis 300 U/ml, bevorzugt 40 bis 150 U/ml Streptokinase enthält.

**9.** Reagenz nach Anspruch 7,
**dadurch gekennzeichnet,**
daß es 10 bis 120 U/ml, bevorzugt 20 bis 80 U/ml Urokinase enthält.

**10.** Reagenz nach Anspruch 7,
**dadurch gekennzeichnet,**
daß es 0,2 bis 10 $\mu$g/ml, bevorzugt 0,6 bis 3 $\mu$g/ml Extrinsic-Plasminogen-Aktivator (EPA) enthält.

**Claims**

**1.** Process for the determination of a partner of an immune reaction in a plasma sample according to the immunoassay principle at a temperature between 15 and 40°C, whereby one of the reaction partners is present in solid phase, characterised in that one adds a plasminogen activator to the incubation medium of the immune reaction.

**2.** Process according to claim 1, characterised in that one uses streptokinase or urokinase as plasminogen activator.

**3.** Process according to claim 2, characterised in that one adds 10 to 300 U/ml, preferably 40 to 150 U/ml of streptokinase.

**4.** Process according to claim 2, characterised in that one adds 10 to 120 U/ml, preferably 20 to 80 U/ml of urokinase.

**5.** Process according to claim 1, characterised in that one adds extrinsic plasminogen activator (EPA) as plasminogen activator.

**6.** Reagent for the carrying out of the process according to one of claims 1 to 5, containing mobilised and immobilised partners of an immune reaction, characterised in that it contains a plasminogen activator.

**7.** Reagent according to claim 6, characterised in that it contains streptokinase, urokinase or extrinsic plasminogen activator (EPA) as plasminogen activator.

**8.** Reagent according to claim 7, characterised in that it contains 10 to 300 U/ml, preferably 40 to 150 U/ml of streptokinase.

**9.** Reagent according to claim 7, characterised in that it contains 10 to 120 U/ml, preferably 20 to 80 U/ml of urokinase.

**10.** Reagent according to claim 7, characterised in that it contains 0.2 to 10 $\mu$g/ml, preferably 0.6 to 3 $\mu$g/ml EPA.

**Revendications**

1. Procédé pour déterminer un partenaire d'une réaction immunitaire dans un échantillon de plasma selon le principe des déterminations immunologiques à une température comprise entre 15 et 40°C, l'un des partenaires de la réaction étant en phase solide, caractérisé en ce que l'on ajoute un activateur du plasminogène au milieu d'incubation de la réaction immunitaire.

2. Procédé selon la revendication 1, caractérisé en ce que l'on utilise comme activateur du plasminogène la streptokinase ou l'urokinase.

3. Procédé selon la revendication 2, caractérisé en ce que l'on ajoute 10 à 300 U/ml, de préférence 40 à 150 U/ml de streptokinase.

4. Procédé selon la revendication 2, caractérisé en ce que l'on ajoute 10 à 120 U/ml, de préférence 20 à 80 U/ml d'urokinase.

5. Procédé selon la revendication 1, caractérisé en ce que l'on ajoute comme activateur du plasminogène l'activateur du plasminogène extrinsèque (EPA).

6. Réactif pour la mise en oeuvre du procédé selon l'une des revendications 1 à 5, contenant des partenaires mobilisés et immobilisés d'une réaction immunitaire, caractérisé en ce qu'il contient un activateur du plasminogène.

7. Réactif selon la revendication 6, caractérisé en ce qu'il contient comme activateur du plasminogène la streptokinase, l'urokinase ou l'activateur du plasminogène extrinsèque (EPA).

8. Réactif selon la revendication 7, caractérisé en ce qu'il contient 10 à 300 U/ml, de préférence 40 à 150 U/ml de streptokinase.

9. Réactif selon la revendication 7, caractérisé en ce qu'il contient 10 à 120 U/ml, de préférence 20 à 80 U/ml d'urokinase.

10. Réactif selon la revendication 7, caractérisé en ce qu'il contient 0,2 à 10 $\mu$g/ml, de préférence 0,6 à 3 $\mu$g/ml d'activateur du plasminogène extrinsèque (EPA).